(19) Europäisches Patentamt
European Patent Office
Office européen des brevets

(11) Veröffentlichungsnummer : **0 025 516**
**B1**

(12) **EUROPÄISCHE PATENTSCHRIFT**

(45) Veröffentlichungstag der Patentschrift : **16.02.83**

(51) Int. Cl.³ : **A 01 N 43/64**, C 07 D249/08

(21) Anmeldenummer : **80104826.5**

(22) Anmeldetag : **14.08.80**

# (54) Triazolylether enthaltende Mittel zur Regulierung des Pflanzenwachstums, Verfahren zur Herstellung der Mittel und Verfahren zur Regulierung des Pflanzenwachstums.

(30) Priorität : **27.08.79 DE 2934507**

(43) Veröffentlichungstag der Anmeldung : **25.03.81 Patentblatt 81/12**

(45) Bekanntmachung des Hinweises auf die Patenterteilung : **16.02.83 Patentblatt 83/07**

(84) Benannte Vertragsstaaten : **AT BE CH DE FR GB IT LI NL SE**

(56) Entgegenhaltungen : **DE A 2 407 143**

(73) Patentinhaber : **BASF Aktiengesellschaft**
**Carl-Bosch-Strasse 38**
**D-6700 Ludwigshafen (DE)**

(72) Erfinder : **Zeeh, Bernd, Dr.**
**Thorwaldsenstrasse 5**
**D-6700 Ludwigshafen (DE)**
Erfinder : **Ammermann, Eberhard, Dr.**
**Sachsenstrasse 3**
**D-6700 Ludwigshafen (DE)**
Erfinder : **Buschmann, Erst, Dr.**
**Georg-Ludwig-Krebs-Strasse 10**
**D-6700 Ludwigshafen (DE)**
Erfinder : **Jung, Johann, Dr.**
**Hardenburgstrasse 19**
**D-6703 Limburgerhof (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Triazolylether enthaltende Mittel zur Regulierung des Pflanzenwachstums, Verfahren zur Herstellung der Mittel und Verfahren zur Regulierung des Pflanzenwachstums

Die vorliegende Erfindung betrifft Triazolylether enthaltende Mittel zur Regulierung des Pflanzenwachstums, Verfahren zur Herstellung dieser Mittel und Verfahren zur Regulierung des Pflanzenwachstums mit den Triazolylethern.

Es ist bekannt, daß 2-Chlorethyltrimethylammoniumchlorid, CCC, (J. Biol. Chem. *235*, 475 (1960) pflanzenwachstumsregulierende Eigenschaften bei Getreide und anderen Kulturpflanzen aufweist (US-Patent 3 156 544). Ebenso ist es bekannt, daß Triazolylalkohole (DE-OS 27 37 489, 24 07 143) das Pflanzenwachstum beeinflussen.

Bei der Anwendung der bekannten Mittel zur Regulierung des Pflanzenwachstums ist die Wirkung vor allem bei niedrigen Aufwandmengen und Konzentrationen oft nicht ausreichend.

Es wurde gefunden, daß Mittel zur Regulierung des Pflanzenwachstums, die Triazolylether der Formel I

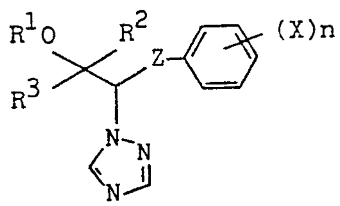

(I)

in der

$R^1$, $R^2$ und $R^3$ einen Alkylrest, einen Alkenylrest oder einen Alkinylrest bedeuten, wobei $R^1$, $R^2$ und $R^3$ jeweils mit 1 bis 3 Halogenatomen substituiert sein können und $R^2$ oder $R^3$ zusätzlich für Wasserstoff steht, in der

X Halogen oder Phenyl,

n die Zahlen 0 bis 3 und

Z $CH_2$ bedeuten oder die pflanzenphysiologisch verträglichen Salze dieser Triazolylether (z. B. die Hydrochloride, Bromide, Sulfate, Nitrate, Oxalate, Acetate, Formiate, Phosphate) enthalten, starke pflanzenwachstumsregulierende Eigenschaften haben und den in den DE-OS 27 37 489 und DE-OS 24 07 143 beschriebenen Substanzen in ihrer Wirkung überlegen sind.

$R^1$, $R^2$ und $R^3$ bedeuten beispielsweise: Methyl, Ethyl, Propyl, n-Butyl, t-Butyl, Isobutyl, 3-Methylbutyl, 2-Methylbutyl, n-Pentyl, Allyl, Crotyl, Methallyl, 3-Methyl-buten-2-yl-1, 1-Buten-4-yl, Propargyl, 2-Butin-1-yl, 2,3-Dichlorallyl, Vinyl.

X bedeutet beispielsweise Chlor, Brom, Jod, Fluor, Phenyl.

Überraschend zeigen die erfindungsgemäßen Mittel eine wesentlich stärkere pflanzenwachstumsregulierende Wirkung als das bekannte 2-Chlorethyl-trimethylammoniumchlorid, welches ein anerkannt gut wirksamer Stoff gleicher Wirkungsart ist.

Die Mittel greifen in den Metabolismus der Pflanzen ein und sind deshalb Wachstumsregulatoren.

Für die Wirkungsweise von Pflanzenwachstumsregulatoren gilt nach der bisherigen Erfahrung, daß ein Wirkstoff eine oder auch mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium des Samens oder der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in gewünschter Weise positiv beeinflussen.

Pflanzenwuchsregulierende Stoffe können zum Beispiel zur Hemmung des vegetativen Pflanzenwachstums eingesetzt werden. Eine derartige Wuchshemmung ist unter anderem bei Gräsern von wirtschaftlichem Interesse, denn durch eine Dämpfung des Graswachstums kann z. B. die Häufigkeit der Grasschnitte in Ziergärten, Park- und Sportanlagen oder an Straßenrändern reduziert werden. Von Bedeutung ist auch die Hemmung des Wuchses von krautigen und holzigen Pflanzen an Straßenrändern und in der Nähe von Überlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverdürzung wird die Gefahr des Umknickens (« Lagerns ») der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung der Kultur, so daß ein Mehrertrag bezogen auf die Bodenfläche erzielt werden kann.

Ein weiterer Mechanismus der Ertragssteigerung mit Wuchshemmern beruht darauf, daß die Nährstoffe in stärkerem Maße der Blüten- und Fruchtbildung zugute kommen, während das vegetative Wachstum eingeschränkt wird.

Mit Wachstumsregulatoren läßt sich häufig auch eine Förderung des vegetativen Wachstums erzielen. Dies ist von großem Nutzen, wenn die vegetativen Pflanzenteile geerntet werden. Eine

Förderung des vegetativen Wachstums kann aber auch gleichzeitig zu einer Förderung des generativen Wachstums führen, so daß z. B. mehr oder größere Früchte zur Ausbildung kommen.

Ertragssteigerungen können in manchen Fällen auch durch einen Eingriff in den pflanzlichen Stoffwechsel erreicht werden, ohne daß sich Änderungen des vegetativen Wachstums bemerkbar machen. Wachstumsregulatoren können ferner eine Veränderung der Zusammensetzung der Pflanzen bewirken, um so eine bessere Qualität der Ernteprodukte herbeizuführen. So ist es beispielsweise möglich, den Gehalt an Zucker in Zuckerrüben, Zuckerrohr, Ananas sowie Zitrusfrüchten zu erhöhen oder den Proteingehalt in Soja oder Getreide zu steigern.

Unter dem Einfluß von Wachstumsregulatoren kann es zur Ausbildung parthenokaper Früchte kommen. Ferner kann das Geschlecht der Blüten beeinflußt werden.

Mit Wachstumsregulatoren läßt sich auch die Produktion oder der Abfluß von sekundären Pflanzenstoffen positiv beeinflussen. Als Beispiel sei die Stimulierung des Latexflusses bei Gummibäumen genannt.

Während des Wachstums der Pflanze kann durch Einsatz von Wachstumsregulatoren auch die seitliche Verzweigung durch eine chemische Brechung der Apikaldominanz vermehrt werden. Daran besteht z. B. Interesse bei der Stecklingsvermehrung von pflanzen. Es ist jedoch auch möglich, das Wachstum der Seitentriebe zu hemmen, z. B. um bei Tabakpflanzen nach der Dekapitierung die Ausbildung von Seitentrieben zu verhindern und damit das Blattwachstum zu fördern.

Unter dem Einfluß von Wachstumsregulatoren kann der Blattbestand von Pflanzen so gesteuert werden, daß ein Entblättern der Pflanzen zu einem gewünschten Zeitpunkt erreicht wird. Eine derartige Entlaubung ist von Interesse, um eine mechanische Beerntung, z. B. bei Wein oder Baumwolle, zu erleichtern oder um die Transpiration zu einem Zeitpunkt herabzusetzen, an dem die Pflanze verpflanzt werden soll.

Durch Einsatz von Wachstumsregulatoren läßt sich der vorzeitige Fruchtfall verhindern. Es ist jedoch auch möglich, den Fruchtfall, — z. B. bei Obst —, im Sinne einer chemischen Ausdünnung bis zu einem bestimmten Ausmaß zu fördern. Wachstumsregulatoren können auch dazu dienen, um bei Kulturpflanzen zum Zeitpunkt der Ernte die zum Ablösen der Früchte erforderliche Kraft zu vermindern, so daß eine mechanische Ernte ermöglicht beziehungsweise die manuelle Ernte erleichtert wird.

Mit Wachstumsregulatoren läßt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Hilfe von Wachstumsregulatoren auch eine zeitliche konzentrierung der Reife erzielt werden. Damit werden die Voraussetzungen dafür geschaffen, daß z. B. bei Tabak, Tomaten oder Kaffee, eine vollständige mechanische oder manuelle Ernte in nur einem Arbeitsgang vorgenommen werden kann.

Durch Anwendung von Wachstumsregulatoren kann auch die Samen- oder Knospenruhe der Pflanzen, also die endogene Jahresrhythmik, beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen.

Mit Wachstumsregulatoren kann auch erreicht werden, daß der Austrieb von Knospen oder die Keimung von Samen verzögert wird, z. B. um in frostgefährdeten Gebieten eine Schädigung durch Spätfröste zu vermeiden.

Wachstumsregulatoren können auch eine Halophilie (Salzverträglichkeit) bei Kulturpflanzen erzeugen. Damit werden die Voraussetzungen dafür geschaffen, daß eine Kultivierung von Pflanzen auf salzhaltigen Böden durchgeführt werden kann.

Mit Wachstumsregulatoren kann auch eine Frost- und Trockenresistenz induziert werden.

Die Triazolylether der Formel I, in der $R^1$, $R^2$, $R^3$, Z, X und n die oben genannten Bedeutungen haben, werden hergestellt durch Umsetzung der teilweise bekannten Triazolylalkohole der Formel II, in der $R^2$, $R^3$, Z, X und n die oben genannten Bedeutungen haben, mit Alkylierungsmitteln $R^1Y$ der Formel III, in der $R^1$ die oben genannten Bedeutungen hat und Y für eine nucleofuge Gruppe, z. B. Cl, Br, J, $OSO_3R$, $CH_3—C_6H_4—SO_3$, $CH_3O—C_6H_4—SO_3$ steht.

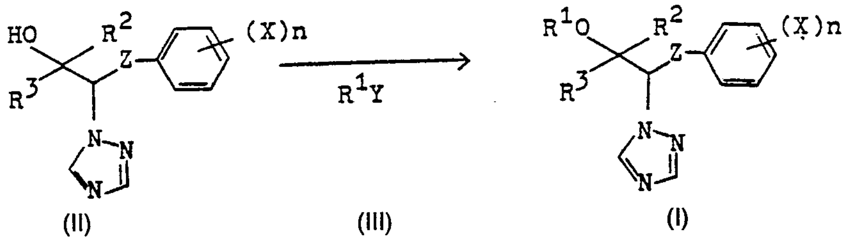

(II) (III) (I)

Die Triazolylalkohole der Formel II sind teilweise bekannt (DE-OS 23 24 010, 27 37 489). Die bisher noch nicht bekannten Verbindungen der Formel II lassen sich nach bekannten Verfahren in einfacher Weise herstellen, indem man beispielsweise die bekannten Triazolylketone der Formel IV (DE-OS 22 01 063, 26 38 470) auf bekannte Weise mit Reduktionsmitteln wie $NaBH_4$, $H_2$/Katalysator umsetzt, oder indem man die Trazolylketone der Formel IV mit Metallalkylen der Formel V zur Reaktion bringt.

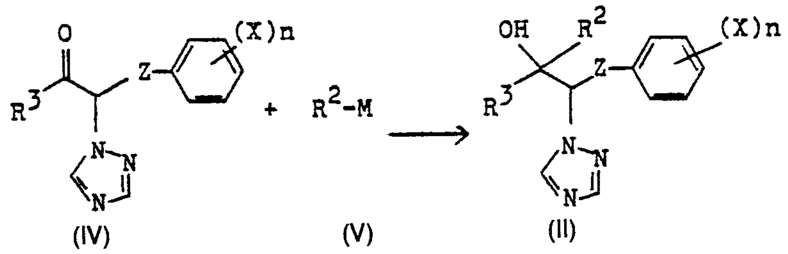

(IV) (V) (II)

Metallalkyle der Formel V sind beispielsweise : Ethylmagnesiumbromid, Methylmagnesiumbromid, Allylmagnesiumbromid, Proparylmagnesiumbromid, Vinylmagnesiumbromid, Ethinylmagnesiumchlorid, Vinylmagnesiumchlorid, Methylmagnesiumjodid, Butyllithium, Natriummethyl. Die Verbindungen der Formel II erhält man, indem man zu einer Lösung der Metallalkylverbindung V in einem geeigneten Lösungsmittel, z. B. Diethylether, Methyl-tert.-butylether, Tetrahydrofuran, Dioxan das Triazolylketon der Formel IV zutropft und anschließend eventuell zum Rückfluß erwärmt.

Beispiele für Verbindungen der Formel II zeigt die folgende Tabelle 1.

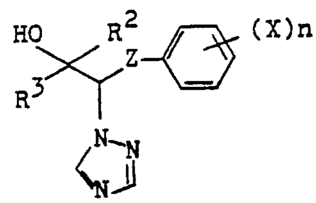

Tabelle 1

| $R^3$ | $R^2$ | Z | (X)n | Schmp. |
|---|---|---|---|---|
| t-Butyl | $-CH_3$ | $CH_2$ | 4-Cl | 176-179 |
| " | $-CH=CH_2$ | $CH_2$ | 4-Cl | |
| " | $-C\equiv CH$ | $CH_2$ | 4-Cl | |
| " | $-C_2H_5$ | $CH_2$ | 4-Cl | |
| " | $CH_2CH=CH_2$ | $CH_2$ | 4-Cl | 148-151 |
| " | $CH_2C\equiv CH$ | $CH_2$ | 4-Cl | |
| " | $nC_4H_9$ | $CH_2$ | 4-Cl | |
| " | $CH_3$ | $CH_2$ | 2,4-Cl | |
| " | $-CH=CH_2$ | $CH_2$ | 2,4-Cl | |
| " | $CH_3$ | $CH_2$ | | |
| " | $-CH=CH_2$ | $CH_2$ | | |
| " | $CH_3$ | $CH_2$ | 4-Br | |
| " | $-CH=CH_2$ | $CH_2$ | 4-Br | |
| " | H | $CH_2$ | 4-Cl | |
| " | H | $CH_2$ | 2,4-Cl | |
| " | H | $CH_2$ | 4-Br | |
| " | H | $CH_2$ | | |

Weitere Ausgangsverbindungen für die Herstellung der Triazolylether der Formel I sind beispielsweise die folgenden Alkylierungsmittel der Formel III : Methylbromid, Methyljodid, Dimethylsulfat, $FSO_3CH_3$, Ethylbromid, Ethyljodid, Allylbromid, Allyljodid, Brompropan, Propylchlorid, Propyljodid, Isobutylbromid, n-Butylbromid, Isobutylbromid, 2-Methyl-butylbromid, 3-Methyl-butylbromid, Pentylbromid, $CH_3—C_6H_4—SO_3CH_3$, $CH_3—C_6H_4—SO_3C_2H_5$.

Die Verbindungen der Formel I erhält man, indem man zu einer Lösung der Verbindungen der Formel II in einem geeigneten Lösungsmittel z. B. Tetrahydrofuran, Dioxan, Methyl-tert.-butylether, Dimethylformamid, Dimethylacetamid in Gegenwart einer geeigneten Base wie Diisopropylethylamin, Lithiumdiisopropylamid, Butyllithium, Natriumhydrid das Alkylierungsmittel der Formel III zutropft und eventuell bei Normaldruck oder erhöhtem Druck erwärmt.

Gut geeignet für die Alkylierung der Alkohole der Formel II ist auch die Phasentransfermethode (E.V. Dehmlow, Angew. Chem. *89*, 521 bis 533 (1977)).

Die folgenden Vorschriften erläutern die Herstellung der Verbindungen der Formel I.

Vorschrift 1

Zu einer Suspension von 1,6 g Natriumhydrid in 80 ml Tetrahydrofuran werden bei 50 bis 60 °C 14,7 g 1-p-Chlorphenyl-4,4-dimethyl-2-(1,2,4-triazol-1-yl)-pentan-3-ol in 70 ml Tetrahydrofuran zugetropft. Man erwärmt 2 Stunden zum Rückfluß, tropft 6,5 g Ethylbromid zu und erwärmt weiter 5 Stunden zum Rückfluß. Nachdem man nochmals 6,5 g Ethylbromid zugegeben und 5 Stunden zum Rückfluß erwärmt hat, werden 150 ml Wasser zugetropft. Man extrahiert dreimal mit je 100 ml Ether. Die vereinigten Etherphasen werden dreimal mit 50 ml Wasser gewaschen, über $Na_2SO_4$ getrocknet und eingeengt. Chromatographie des öligen Rohprodukts über Kieselgel mit $CH_2Cl_2$ ergibt 6,5 g 1-p-Chlorphenyl-4,4-dimethyl-3-ethoxy-2-(1,2,4-triazol-1-yl)-pentan. Schmp. 106 bis 108 °C (Wirkstoff Nr. 2).

Vorschrift 2

Zu einer Suspension von 1,6 g Natriumhydrid in 100 ml Tetrahydrofuran wird zugetropft eine Lösung von 15,4 g 1-p-Chlorphenyl-3,4,4-trimethyl-2-(1,2,4-triazol-1-yl)-pentan-3-ol in 100 ml Tetrahydrofuran. Man erwärmt 2 Stunden zum Rückfluß. Nach Abkühlen werden 8,5 g-Jodmethan zugetropft. Man erwärmt 5 Stunden zum Rückfluß, gibt 200 ml Wasser zu, extrahiert dreimal jeweils 100 ml Ether, wäscht die vereinigten organischen Phasen dreimal mit 50 ml Wasser, trocknet und engt ein. Aus Petroläther kristallisieren 9,5 g 1-p-Chlorphenyl-3,4,4-trimethyl-3-methoxy-2-(1,2,4-triazol-1-yl)-pentan, Schmp. 125 bis 127 °C (Wirkstoff Nr. 15).

In analoger Weise sind die Verbindungen der Tabelle 2 zugänglich.

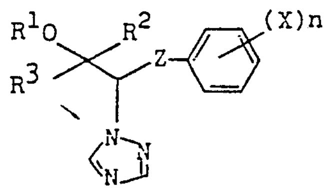

Tabelle 2

| Wirkstoff Nr. | $R^1$ | $R^2$ | $R^3$ | Z | (X)n | Schmp. °C |
|---|---|---|---|---|---|---|
| 1 | $CH_3$ | H | t-Bu | $CH_2$ | 4-Cl | |
| 2 | $C_2H_5$ | H | t-Bu | $CH_2$ | 4-Cl | 106-108 |
| 3 | $n-C_3H_7$ | H | t-Bu | $CH_2$ | 4-Cl | |
| 4 | $nC_4H_9$ | H | t-Bu | $CH_2$ | 4-Cl | |
| 5 | $CH_2CH=CH_2$ | H | t-Bu | $CH_2$ | 4-Cl | 105-107 |
| 6 | $CH_2C≡CH$ | H | t-Bu | $CH_2$ | 4-Cl | 98-100 |
| 7 | $CH_2CCl=CHCl$ | H | t-Bu | $CH_2$ | 4-Cl | |
| 8 | $CH_3$ | H | t-Bu | $CH_2$ | $2,4-Cl_2$ | |
| 9 | $CH_2CH=CH_2$ | H | t-Bu | $CH_2$ | $2,4-Cl_2$ | 90-95 |
| 10 | $CH_2C≡CH$ | H | t-Bu | $CH_2$ | $2,4-Cl_2$ | |
| 11 | $CH_3$ | H | t-Bu | $CH_2$ | | |
| 12 | $CH_2CH=CH_2$ | H | t-Bu | $CH_2$ | | |
| 13 | $nC_4H_9$ | H | t-Bu | $CH_2$ | | |
| 14 | $CH_3$ | H | t-Bu | $CH_2$ | 4-Br | |
| 15 | $CH_3$ | $CH_3$ | t-Bu | $CH_2$ | 4-Cl | 125-127 |
| 16 | $C_2H_5$ | $CH_3$ | t-Bu | $CH_2$ | 4-Cl | |
| 17 | $CH_2CH=CH_2$ | $CH_3$ | t-Bu | $CH_2$ | 4-Cl | |
| 18 | $CH_2C≡CH$ | $CH_3$ | t-Bu | $CH_2$ | 4-Cl | |
| 19 | $CH_3$ | $-CH=CH_2$ | t-Bu | $CH_2$ | 4-Cl | |

Tabelle 2 (Fortsetzung)

| Wirkstoff Nr. | $R^1$ | $R^2$ | $R^3$ | Z | (X)n - | Schmp. °C |
|---|---|---|---|---|---|---|
| 20 | $C_2H_5$ | $-CH=CH_2$ | t-Bu | $CH_2$ | 4-Cl | |
| 21 | $CH_2CH=CH_2$ | $-CH=CH_2$ | t-Bu | $CH_2$ | 4-Cl | |
| 22 | $CH_2C\equiv CH$ | $-CH=CH_2$ | t-Bu | $CH_2$ | 4-Cl | |
| 23 | $CH_3$ | $-C\equiv CH$ | t-Bu | $CH_2$ | 4-Cl | |
| 35 | $C_2H_5$ | H | t-Bu | $CH_2$ | $2,4-Cl_2$ | 102-104 |
| 36 | $CH_3$ | H | t-Bu | $CH_2$ | $C_6H_5$ | |
| 37 | $CH_2CH=CH_2$ | H | t-Bu | $CH_2$ | $C_6H_5$ | |
| 38 | $CH_3$ | $CH_3$ | t-Bu | $CH_2$ | $C_6H_5$ | |
| 39 | $CH_2CH=CH_2$ | $CH_3$ | t-Bu | $CH_2$ | $C_6H_5$ | |

t-Bu = tertiär-Butyl

Die neuen Mittel können in üblichen Formulierungen vorliegen, wie Lösungen, Emulsionen, Suspensionen, Stäube, Pulver, Pasten und Granulate. Die Anwendungsformen richten sich ganz nach den Verwendungszwecken ; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage : Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin, Dimethylformamid) und Wasser ; Trägerstoffe wie natürliche Gesteinsmehle (z. B. Kaoline, Tonerden, Talkum, Kreide) und synthetische Gesteinsmehle (z. B. hochdisperse Kieselsäure, Silikate) : Emulgiermittel wie nichtionogene und anionische Emulgatoren (z. B. Polyoxyethylen — Fettalkohol — Ether, Alkylsulfonate und Arylsulfonate) und Dispergiermittel, wie Lignin, Sulfitablaugen und Methylcellulose.

Die Mittel enthalten im allgemeinen zwischen 0,1 und 95 Gew.-% Wirkstoff, vorzugsweise zwischen 0,5 und 90.

Die Aufwandmengen liegen nach Art des gewünschten Effektes zwischen 0,01 und 3, vorzugsweise jedoch zwischen 0,01 und 1 kg Wirkstoff pro Hektar.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind :

I. Man vermischt 90 Gewichtsteile des Wirkstoffs 1 mit 10 Gewichtsteilen N-Methyl-α-pyrrolidon und erhält eine Lösung, die zur Anwendung in Form kleinster Tropfen geeignet ist.

II. 20 Gewichtsteile des Wirkstoffs 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100.000 Gewichtsteilen Wasser erhält man eine wäßrige Dispersion, die 0,02 Gew.% des Wirkstoffs enthält.

III. 20 Gewichtsteile des Wirkstoffs 3 werden in einer Mischung gelöst, die aus 40 Gewichtsteilen Cyclohexan, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in 100.000 Gewichtsteile 0,02 Gew.% des Wirkstoffs enthält.

IV. 20 Gewichtsteile des Wirkstoffs 4 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100.000 Gewichtsteilen Wasser erhält man eine wäßrige Dispersion, die 0,02 Gew.% des Wirkstoffs enthält.

V. 20 Gewichtsteile des Wirkstoffs 1 werden den mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselgel gut vermischt und in einer

Hammermühle vermahlen. Durch feines Verteilen der Mischung in 20.000 Gewichtsteilen Wasser erhält man eine Spritzbrühe, die 0,1 Gew.% des Wirkstoffs enthält.

VI. 3 Gewichtsteile des Wirkstoffs 2 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile des Wirkstoffs 3 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile des Wirkstoffs 4 werden mit 10 Teilen Natriumsalz eines Phenolsulfonsäureharnstoffformaldehyd-Kondensates, 2 Teilen Kieselgel und 48 Teilen Wasser innig vermischt. Man erhält eine stabile wäßrige Dispersion. Durch Verdünnen mit 100.000 Gewichtsteilen erhält man eine wäßrige Dispersion, die 0,04 Gew.% Wirkstoff enthält.

IX. 20 Teile des Wirkstoffs 1 werden mit 2 Teilen Calciumsalz der Dodecylbenzolsulfonsäure, 8 Teilen Fettalkoholpolyglykolether, 2 Teilen Natriumsalz eines Phenolsulfonsäureharnstoff-formaldehyd-Kondensates und 68 Teilen eines paraffinischen Mineralöls innig vermischt. Man erhält eine stabile öblige Dispersion.

Die erfindungsgemäßen Mittel können in diesen Anwendungsformen auch zusammen mit anderen Wirkstoffen vorliegen, wie z. B. Herbiziden, Insektiziden, Wachstumsregulatoren und Fungiziden, oder auch mit Düngemitteln vermischt und ausgebracht werden.

Zur Bestimmung der wachstumregulierenden Eigenschaft der Prüfsubstanzen wurden Testpflanzen auf ausreichend mit Nährstoffen versorgtem Kultursubstrat in Kunststoffgefäßen von ca. 12,5 cm Durchmesser angezogen.

Die zu prüfenden Substanzen Wurden in wäßriger Aufbereitung auf die Pflanzen gesprüht (Nachauflaufverfahren). Die beobachtete wachstumsregulierende Wirkung wurde bei Versuchsende durch Wuchshöhenmessung bestimmt. Die so gewonnenen Meßwerte wurden zur Wuchshöhe der unbehandelten Pflanzen in Relation gesetzt. Als Vergleichssubstanz diente CCC.

Gleichlaufend mit der Reduzierung des Längenwachstums stieg die Farbintensität der Blätter an. Der erhöhte Chlorophyllgehalt läßt eine ebenfalls erhöhte Photosynthese und damit einen erhöhten Ertrag erwarten.

Die Einzeldaten sind den folgenden Tabellen zu entnehmen.

Vergleichssubstanz :

CCC $$CH_3-\overset{CH_3}{\underset{CH_3}{N^{\oplus}}}-CH_2-CH_2-Cl \qquad Cl^{\ominus}$$

Sommerraps « Cosa »

Nachauflaufverfahren ; Versuchsdauer : 20 Tage

| Wirkstoff Nr. | mg Wirkstoff/Gef. | Wuchshöhen cm | % |
|---|---|---|---|
| unbehandelt | - | 20,7 | 100 |
| CCC (bekannt) | 1,5 | 19,0 | 91,8 |
| | 6 | 19,0 | 91,8 |
| 5 | 1,5 | 15,5 | 74,9 |
| | 6 | 14,0 | 67,6 |
| 6 | 1,5 | 16,5 | 79,7 |
| | 6 | 13,0 | 62,8 |

Tomaten « Rotkäppchen »

Nachauflaufverfahren ; Versuchsdauer : 45 Tage

| Wirkstoff Nr. | mg Wirkstoff/Gef. | Wuchshöhen cm | % |
|---|---|---|---|
| unbehandelt | - | 23 | 100 |
| CCC (bekannt) | 1,5 | 23 | 100 |
| | 6 | 21,4 | 92,9 |
| 2 | 1,5 | 16,0 | 69,6 |
| | 6 | 15,3 | 66,5 |

Tomaten « Sonata »

Nachauflaufverfahren : Versuchsdauer : 50 Tage

| Wirkstoff Nr. | mg Wirkstoff/Gef. | Wuchshöhen cm | % |
|---|---|---|---|
| unbehandelt | - | 34,6 | 100 |
| CCC (bekannt) | 1,5 | 33,6 | 97,0 |
| | 6 | 33,3 | 96,3 |
| 5 | 1,5 | 33,8 | 97,7 |
| | 6 | 25,0 | 72,3 |
| 6 | 1,5 | 29,0 | 83,8 |
| | 6 | 25,5 | 73,7 |

Salat « Lüneburger Eis »

Nachauflaufverfahren ; Versuchsdauer : 26 Tage

| Wirkstoff Nr. | mg Wirkstoff/Gef. | Wuchshöhen cm | % |
|---|---|---|---|
| unbehandelt | - | 18,5 | 100 |
| CCC (bekannt) | 0,75 | 17,5 | 94,6 |
| | 1,5 | 17,0 | 91,9 |
| 2 | 0,75 | 14,5 | 78,4 |
| | 1,5 | 14,0 | 75,7 |
| 5 | 0,75 | 12,5 | 67,6 |
| | 1,5 | 12,3 | 66,5 |
| 6 | 0,75 | 13,3 | 71,9 |
| | 1,5 | 10,8 | 58,4 |

**Ansprüche**

1. Mittel zur Regulierung des Pflanzenwachstums, gekennzeichnet durch einen Gehalt an einem Triazolylether der Formel I,

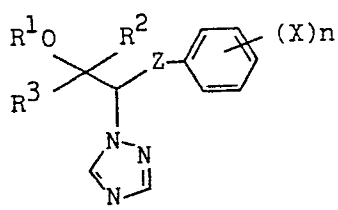

(I)

in der

$R^1$, $R^2$ und $R^3$ Alkyl-, Alkenyl- oder Alkinylreste bedeuten, die mit 1 bis 3 Halogenatomen substitutiert sein können, wobei $R^2$ oder $R^3$ auch für Wasserstoff stehen kann, in der

X Halogen oder Phenyl

n die Zahlen 0 bis 3 und

Z $CH_2$ bedeuten oder einem pflanzenphysiologisch verträglichen Salz dieser Verbindungen.

2. Mittel zur Regulierung des Pflanzenwachstums enthaltend einem festen oder flüssigen Trägerstoff und einen Triazolylether der Formel

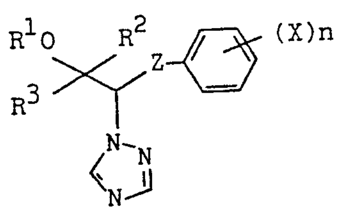

(I)

in der

$R^1$, $R^2$ und $R^3$ Alkyl-, Alkenyl- oder Alkinylreste bedeuten, die mit 1 bis 3 Halogenatomen substitutiert sein können, wobei $R^2$ oder $R^3$ auch für Wasserstoff stehen kann, in der

X Halogen oder Phenyl

n die Zahlen 0 bis 3 und

Z $CH_2$ bedeuten oder einem pflanzenphysiologisch verträglichen Salz dieser Verbindungen.

3. Verfahren zur Herstellung eines Mittels zur Regulierung des Pflanzenwachstums, dadurch gekennzeichnet, daß man einen festen oder flüssigen Trägerstoff vermischt mit einem Triazolylether der Formel

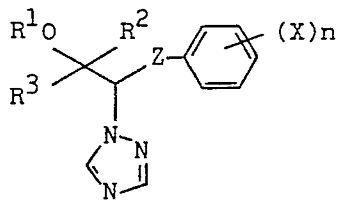

(I)

in der

$R^1$, $R^2$ und $R^3$ Alkyl-, Alkenyl- oder Alkinylreste bedeuten, die mit 1 bis 3 Halogenatomen substituiert sein können, wobei $R^2$ oder $R^3$ auch für Wasserstoff stehen kann, in der

X Halogen oder Phenyl

n die Zahlen 0 bis 3 und

Z $CH_2$ bedeuten oder einem pflanzenphysiologisch verträglichen Salz dieser Verbindungen.

4. Verfahren zur Regulierung des Pflanzenwachstums, dadurch gekennzeichnet, daß man den Boden, die Pflanzen oder ihren Samen behandelt mit einem Triazolylether der Formel

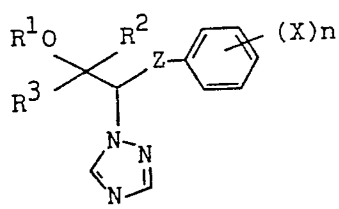

(I)

in der

$R^1$, $R^2$ und $R^3$ Alkyl-, Alkenyl- oder Alkinylreste bedeuten, die mit 1 bis 3 Halogenatomen substituiert sein können, wobei $R^2$ oder $R^3$ auch für Wasserstoff stehen kann, in der

X Halogen oder Phenyl

n die Zahlen 0 bis 3 und

Z $CH_2$ bedeuten oder einem pflanzenphysiologisch verträglichen Salz dieser Verbindungen.

5. Mittel zur Regulierung des Pflanzenwachstums enthaltend einen Triazolylether ausgewählt aus der Gruppe bestehend aus

1-p-Chlorphenyl-4,4-dimethyl-3-allyloxy-2-(1,2,4-triazol-1-yl)-pentan,

1-p-Chlorphenyl-4,4-dimethyl-3-propinoxy-2-(1,2,4-triazol-1-yl)-pentan,

1-p-Chlorphenyl-4,4-dimethyl-3-methoxy-2-(1,2,4-triazol-1-yl)-pentan,

1-p-Chlorphenyl-4,4-dimethyl-3-ethoxy-2(1,2,4-triazol-1-yl)-pentan,

## Claims

1. An agent for regulating plant growth, characterized by the content of a triazolyl ether of the formula

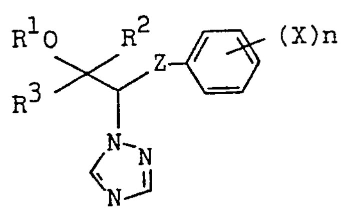

(I)

where

$R^1$, $R^2$ and $R^3$ denote alkyl, alkenyl or alkynyl, each of which is unsubstituted or substituted by from 1

to 3 halogen atoms, and $R^2$ and $R^3$ may additionally denote hydrogen,

X denotes halogen or phenyl,

n denotes the integers 0 to 3, and

Z denotes $CH_2$, or a salt thereof which is tolerated by plants.

2. An agent for regulating plant growth, containing a solid or liquid carrier and a triazolyl ether of the formula

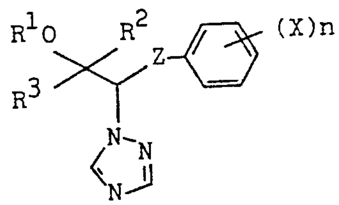

(I)

where

$R^1$, $R^2$ and $R^3$ denote alkyl, alkenyl or alkynyl, each of which is unsubstituted or substituted by from 1 to 3 of which is unsubstituted or substituted by from 1 to 3 halogen atoms, and $R^2$ and $R^3$ may additionally denote hydrogen,

X denotes halogen or phenyl,

n denotes the integers 0 to 3, and

Z denotes $CH_2$, or a salt thereof which is tolerated by the plants.

3. A process for preparing an agent for regulating plant growth, wherein a solid or liquid carrier is mixed with a triazolyl ether of the formula

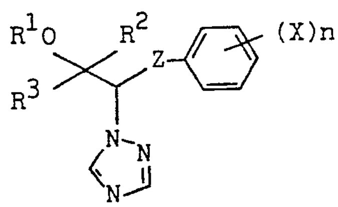

(I)

where

$R^1$ and $R^3$ denote alkyl, alkenyl or alkynyl, each of which is unsubstituted or substituted by from 1 to 3 halogen atoms, and $R^2$ and $R^3$ may additionally denote hydrogen,

X denotes halogen or phenyl,

n denotes the integers 0 to 3, and

Z denotes $CH_2$, or a salt thereof which is tolerated by plants.

4. A process for regulating plant growth, wherein the soil, the plants or their seed are treated with a triazolyl ether of the formula

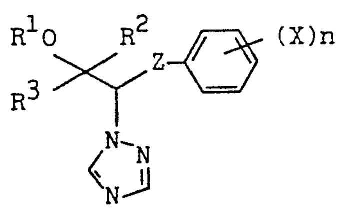

(I)

where

$R^1$, $R^2$ and $R^3$ denote alkyl, alkenyl or alkynyl, each of which is unsubstituted or substituted by from 1 to 3 halogen atoms, and $R^2$ and $R^3$ may additionally denote hydrogen,

X denotes halogen or phenyl,

n denotes the integers 0 to 3 and

Z denotes $CH_2$ or a salt thereof which is tolerated by the plants.

5. An agent for regulating plant growth, containing a triazolyl ether selected from the group consisting of

1-p-chlorophenyl-4,4-dimethyl-3-allyloxy-2-(1,2,4-triazol-1-yl)-pentane,

1-p-chlorophenyl-4,4-dimethyl-3-propynoxy-2-(1,2,4-triazol-1-yl)-pentane,

1-p-chlorophenyl-4,4-dimethyl-3-methoxy-2-(1,2,4-triazol-1-yl)-pentane, and

1-p-chlorophenyl-4,4-dimethyl-3-ethoxy-2-(1,2,4-triazol-1-yl)-pentane.

**Revendications**

1. Moyen de régularisation de la croissance des plantes, caractérisé par une teneur en un triazolyléther de formule I

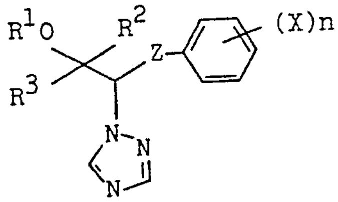

(I)

dans laquelle
$R^1$, $R^2$ et $R^3$ représentent des restes alkyle, alcényle ou alcinyle, qui peuvent être substitués par 1 à 3 atomes d'halogène, $R^2$ ou $R^3$ pouvant être aussi l'hydrogène,
X représente halogène ou phényle,
n un nombre de 0 à 3, et
Z représente $CH_2$ ou un sel de ces composés, compatible au point de vue physiologie des plantes.

2. Moyen de régularisation de la croissance des plantes, contenant un support solide ou liquide et un triazolyléther de formule I

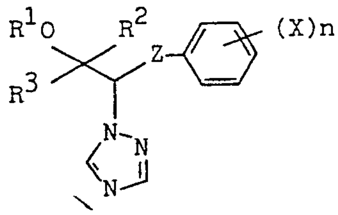

(I)

dans laquelle
$R^1$, $R^2$ et $R^3$ représentent des restes alkyle, alcényle ou alcinyle, qui peuvent être substitués par 1 à 3 atomes d'halogène, $R^2$ ou $R^3$ pouvant être aussi l'hydrogène,
X représente halogène ou phényle,
n un nombre de 0 à 3, et
Z représente $CH_2$ ou un sel de ces composés, compatible au point de vue physiologie des plantes.

3. Procédé de préparation d'un moyen de régularisation de la croissance des plantes, caractérisé par le fait qu'on mélange un support solide ou liquide avec un triazolyléther de formule I

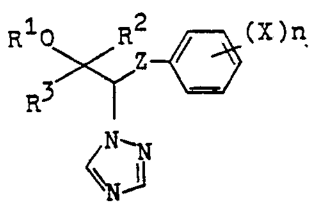

(I)

dans laquelle
$R^1$, $R^2$ et $R^3$ représentent des restes alkyle, alcényle ou alcinyle, qui peuvent être substitués par 1 à 3 atomes d'halogène, $R^2$ ou $R^3$ pouvant être aussi l'hydrogène,
X représente halogène ou phényle,
n un nombre de 0 à 3, et
Z représente $CH_2$ ou un sel de ces composés, compatible au point de vue physiologie des plantes.

4. Procédé de préparation d'un moyen de régularisation de la croissance des plantes, caractérisé par le fait qu'on traite le sol, les plantes ou leurs graines avec un triazolyléther de formule I

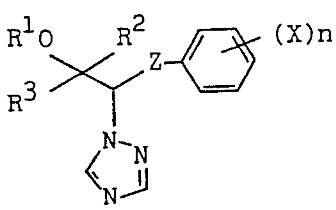

(I)

dans laquelle

$R^1$, $R^2$ et $R^3$ représentent des restes alkyle, alcényle ou alcinyle, qui peuvent être substitués par 1 à 3 atomes d'halogène, $R^2$ ou $R^3$ pouvant être aussi l'hydrogène,

X représente halogène ou phényle,

n un nombre de 0 à 3, et

Z représente $CH_2$ ou un sel de ces composés, compatible au point de vue physiologie des plantes.

5. Moyen de régularisation de la croissance des plantes, contenant un triazolyléther choisi dans le groupe constitué de

1-p-chlorophényl-4,4-diméthyl-3-allyloxy-2-(1,2,4-triazol-1-yl)-pentane,

1-p-chlorophényl-4,4-diméthyl-3-propinoxy-2-(1,2,4-triazol-1-yl)-pentane,

1-p-chlorophényl-4,4-diméthyl-3-méthoxy-2-(1,2,4-triazol-1-yl)-pentane,

1-p-chlorophényl-4,4-diméthyl-3-éthoxy-2-(1,2,4-triazol-1-yl)-pentane.